(19) Europäisches Patentamt
European Patent Office
Office européen des brevets

(11) Publication number: **0 002 586**
**B1**

(12) # EUROPEAN PATENT SPECIFICATION

(45) Date of publication of patent specification: **20.01.82**

(51) Int. Cl.³: **C 07 D 501/20,** C 07 D 277/40, C 07 C 87/60

(21) Application number: **78300765.1**

(22) Date of filing: **07.12.78**

(54) **Method for removing a halogenoacetyl protective group from a halogenoacetyl amino compound.**

(30) Priority: **09.12.77 JP 148414/77**

(43) Date of publication of application:
**27.06.79 Bulletin 79/13**

(45) Publication of the grant of the European patent:
**20.01.82 Bulletin 82/3**

(84) Designated Contracting States:
**CH DE FR GB IT**

(56) References cited:
**BE - A - 852 971**

(73) Proprietor: **Takeda Chemical Industries, Ltd.**
**27, Doshomachi 2-Chome**
**Higashi-ku Osaka, 541 (JP)**

(72) Inventor: **Michihiko, Ochiai**
**23-13, Senriyama-higashi 1-chome, Suita**
**Osaka 565 (JP)**
Inventor: **Akira, Morimoto**
**7-8, Ueikeda 1-chome**
**Ikeda Osaka 563 (JP)**
Inventor: **Toshio, Miyawaki**
**17-27, Hinoikecho**
**Nishinomiya Hyogo 662 (JP)**

(74) Representative: **Lewin, John Harvey et al,**
**Elkington and Fife High Holborn House 52/54**
**High Holborn**
**London WC1V 6SH (GB)**

**EP 0 002 586 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Method for removing a halogenoacetyl protective group from a halogenoacetyl amino compound

The present invention relates to an improved method for removing protecting groups.

More particularly, the improvement lies in employing an N-substituted dithiocarbamic acid or a salt thereof in place of thiourea in the production of an amino compound by removing a halogenoacetyl group from the corresponding halogenoacetyl amino compound.

In the field of chemical reactions, it is common practice, when there is a risk of an amino group of a starting material undergoing undesirable attack by another reagent, to protect the amino group with a suitable protective group prior to subjecting the starting material to an intended reaction. After completion of the reaction, the protective group is removed to obtain the desired amino compound.

As one of such protective groups, halogenoacetyl has been frequently employed. However, removal of the halogenoacetyl group requires relatively severe conditions. For example, treatment with lON-hydrochloric acid and glacial acetic acid [Chemical Abstracts *50,* 15419h (1956)] or treatment with alcoholic HCl [Zeitschrift für Naturforschung *6b,* 340 (1951)] has been conventionally practised. These treatments are disadvantageous in that, were an unstable group exists in the substrate molecule, the compound cannot be safely subjected to such severe conditions. It has recently been discovered that if a halogenoacetyl amino compound is reacted with thiourea, the halogenoacetyl group may be removed by amidinolysis under milder conditions, thus showing that the process is applicable to unstable compounds [J. Am. Chem. Soc. *90,* 4508(1968); J. Chem. Soc. *1965,* 5015]. However, this removal reaction using thiourea has the disadvantage that, in not a few cases, the S-substituted thioformamidine hydrochloride

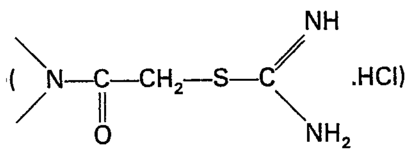

produced as a reaction intermediate must be heated in the presence of water in order to convert the intermediate into the desired amino compound, which has an undesirable effect on the desired amino compound. The reaction of the halogenoacetyl amino compound and thiourea forms the desired amino compound as well as 2-iminothiazolidone through intramolecular amidinolysis. In cases where the solubility of the desired amino compound is similar to that of 2-iminothiazolidone, it is quite difficult to separate the two compounds from each other.

To overcome the shortcomings of the prior art, we have unexpectedly found that removal of the protective group in the present invention proceeds in a shorter reaction time than the known method using thiourea.

We have also found that the solubility of N-substituted rhodanine (i.e. a byproduct corresponding to 2-iminothiazolidone) may be modified by changing the nature of the substituent of the N-substituted dithiocarbamate, taking the solubility of the desired amino compound into consideration. Therefore, the present invention facilitates the recovery of the desired amino compound from the reaction mixture.

More fundamentally, the N-substituted rhodanine is generally soluble in organic solvents and the desired amino compound, particularly a cephem or penam compound having an amino group, is soluble in water. Therefore, the amino compound can be easily separated from the byproduct.

The present invention thus provides an industrially feasible method for producing an amino compound by removing a halogenoacetyl group from a halogenoacetyl amino compound.

The amino compound is intended to mean an organic compound containing one or more amino groups in the molecule.

Examples of the amino compound include aliphatic amino compounds, aromatic amino compounds, five- or six-membered hetero-aromatic amino compounds, alicyclic amino compounds, amino acids, carbamoyl compounds, etc. In other words, the amino compound is exemplified by straight-chain alkanes, branched or straight-chain alkenes, saturated or unsaturated cycloalkanes, aromatic hydrocarbons, or 5- or 6-membered heterocyclic compounds e.g. furan, thiophene, pyrroline, thiazole, oxazole, thiadiazole, oxadiazole, diazole, triazole, tetrazole, pyridine, pyrimidine, pyridazine, which have amino groups as substituents. In addition to one or more amino groups, these compounds may have one or more groups inert to the reaction such as hydroxyl, carboxyl, nitro, halogen, nitroso, alkyl, alkenyl, cycloalkyl, aryl, aralkyl and heterocyclic groups. The method according to this invention is particularly suitable for amino compounds containing a cephem or penam ring, which is comparatively unstable. Such compounds are those of the formulae:

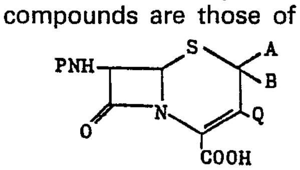

(Cephem Compound)

and

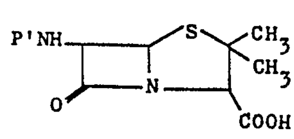

(Penam Compound)

as well as their pharmaceutically acceptable salts, in which each of A and B is hydrogen or methyl, each of P and P′ is an organic acyl group such that the compound constitutes a cephalosporin antibiotic or a penicillin antibiotic, Q is hydrogen or a substituent such that the compound constitutes a cephalosporin antibiotic, and at least one of P and Q has an amino group as a substituent and P′ has an amino group as a substituent.

In the above general formulae, P is exemplified by 2-(2-aminothiazol-4-yl)-2-(*syn*)-methoximino-acetyl, α-aminophenylacetyl, α-sulpho-*p*-aminophenylacetyl, α-amino-*p*-hydroxyphenylacetyl P′ is exemplified by a α-aminophenylacetyl, α-sulpho-p-aminophenylacetyl, α-amino-*p*-hydroxyphenyl-acetyl.

Q is exemplified by hydrogen, (1-methyl-1H-tetrazol-5-yl)thiomethyl, carbamoyloxymethyl, acetoxymethyl, methyl, 5-acetylamino-1,3,4-thiadiazol-2-yl, (2-methylthiadiazol-5-yl)thiomethyl, hydroxymethyl, methoxy and (1-carboxymethyl-1H-tetrazol-5-yl)thiomethyl. Examples of such amino-containing cephem compound include 7-amino-3-carbamoyl-oxymethyl-3-cephem-4-carboxylic acid, 7-[2-(2-aminothiazol-4-yl)-2-(*syn*)-methoxyiminoacetamido]-3-carbamoyloxymethyl-3-cephem-4-carboxylic acid, 7-(5-carboxy-5-aminovaleramido)-3-carbamoyloxymethyl)-3-cephem-4-carboxylic acid, 7-[2-(2-aminothiazol-4-yl)-2-(*syn*)-methoxyiminoacetamido] cephalosporanic acid, 7-[2-(2-amino-thiazol-4-yl)-2-(*syn*)-methoxyiminoacetamido]-3-(1-methyl-1H - tetrazol-5-yl)thiomethyl-3-cephem-4-carboxylic acid, 7-[2-(2-aminothiazol-4-yl)-2-(*syn*)-methoxyiminoacetamido]desacetoxycephalosporanic acid, benzhydryl 7-(2-thienylacetylamido)-3-carbamoyloxymethyl-3-cephem-4-carboxylate, 7-[2-(2-aminothiazol-4-yl)-2-(*syn*)-methoxyiminoacetamido]-3-cephem-4-carboxylic acid, as well as their pharmaceutically acceptable salts. The pharmaceutically acceptable salts are exemplified by alkali metal (e.g. sodium) salts, organic amine (e.g. triethylamine) salts or other salts in cephalosporin antibiotics and penicillin antibiotics.

According to the present invention, the protective group, i.e. a halogenoacetyl group, is removed from a halogenoacetyl amino compound to produce the desired amino compound.

The halogenoacetyl group is exemplified by chloroacetyl, bromoacetyl, fluoroacetyl, but chloroacetyl and bromoacetyl are preferred.

The starting material of the present invention (i.e. a halogenoacetyl amino compound) is a compound having a halogenoacetyl amino group, $XCH_2CO—NH—$ in which X is a halogen, in the molecule, i.e. an amino compound of which the amino group is protected by a halogenoacetyl radical.

The other reagent in the reaction is an N-substituted dithiocarbamic acid of the formula:

$$R'NH\overset{\displaystyle S}{\overset{\|}{C}}SH$$

or a salt thereof, and the salt is preferred for the reaction of the present invention.

The substituent R′ is exemplified by a lower alkyl group with 1 to 6 carbon atoms (such as methyl, ethyl, *n*-propyl, *n*-butyl); a cycloalkyl group with 5 to 7 carbon atoms (such as cyclohexyl, methylcyclohexyl); an aralkyl group with 7 to 10 carbon atoms (such as benzyl, methoxybenzyl, methylbenzyl); and an aryl group of 6 to 10 carbon atoms (such as phenyl, tolyl, methoxyphenyl).

The salt of the N-substituted dithiocarbamic acid is exemplified by a corresponding alkali metal salt, alkaline earth metal salt, ammonia salt and organic amine salt. The alkali metal includes sodium, potassium, the alkaline earth metal includes calcium, magnesium, and the organic amine includes trimethylamine, mono-methylamine, triethylamine, diethylamine, monoethylamine, *n*-propylamine, cyclohexylamine, benzylamine, aniline, N,N-dimethylaniline.

According to the present invention, for example, 7-[2-(2-chloroacetamidothiazol-4-yl)-2-(*syn*)-methoxyiminoacetamido]-3-(1-methyl-1H-tetrazol-5-yl)thiomethyl-3-cephem-4-carboxylic acid or an alkali metal or amine salt thereof is allowed to react with an N-methyl (or ethyl)-dithiocarbamic acid alkali metal salt, or 2-(2-chloroacetamidothiazol-4-yl)-2-(*syn*)-methoxyimino acetic acid or an alkali metal or amine salt thereof is allowed to react with an N-methyl (or ethyl) dithiocarbamic acid alkali metal salt.

The reaction according to this invention proceeds as follows:

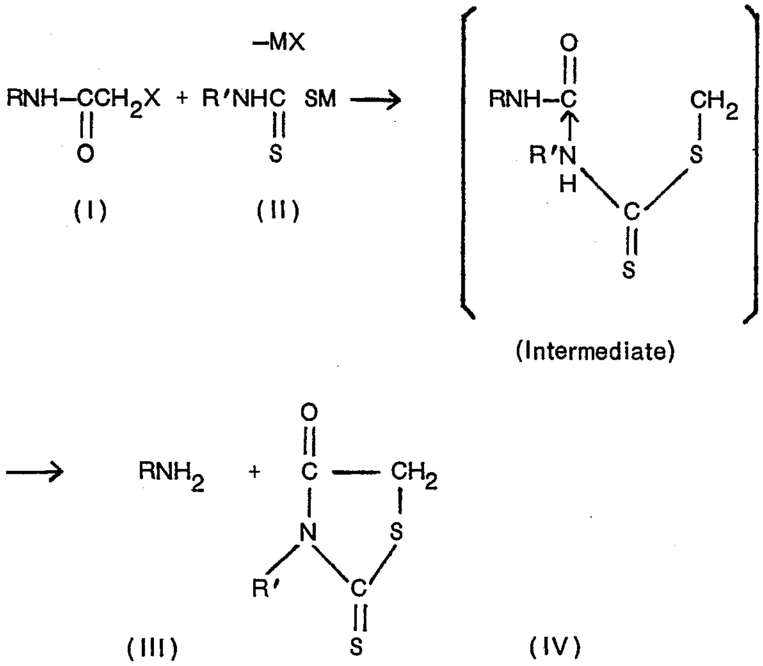


(I) (II) (Intermediate) (III) (IV)

where R is a residue of an amino compound, X is a halogen atom, and M is a salt-forming species.

Compound (I) : a halogenoacetyl amino compound
Compound (II) : an N-substituted dithiocarbamic acid or its salt
Compound (III) : a desired amino compound
Compound (IV) : an N-substituted rhodanine (byproduct)

This reaction is carried out by reacting a compound (I) having a halogenoacetyl amino group with an N-substituted dithiocarbamic acid or a salt thereof (II). The reaction may generally be carried out smoothly in a solvent which may be any optional solvent provided it does not adversely affect the reaction. Thus, suitable solvents are, for example, water, alcohols such as methanol, ethanol, propanol, ethers such as diethyl ether, tetrahydrofuran, dioxane, halogenated hydrocarbons such as chloroform, methylene chloride, dichloroethylene, dichloroethane, and esters such as ethyl acetate, butyl acetate, dimethylsulphoxide, N,N-dimethyl-formamide, N,N-dimethylacetamide, as well as mixtures of such solvents.

The amount of the compound (II) relative to the compound (I) is optional but it is usually in the range of from 0.5 to 3 mols, preferably from 0.8 to 1.3 mols, per mol of the compound (I).

The reaction temperature is usually in the range of from −20°C to 100°C, preferably in the range of from −10°C to 50°C.

The byproduct, N-substituted rhodanine derivative (IV) formed by a kind of aminolysis is a substance generally soluble in organic solvents and can be easily separated from the reaction mixture. The amino-compound ($RNH_2$) obtained on removal of the protective group is isolated and purified by procedures known in the art of general organic chemistry, such as extraction, crystallization, distillation, chromatography, etc.

The N-substituted dithiocarbamic acid or salt which is employed in the reaction according to this invention can be produced by known processes or any process analogous thereto. [cf. Rodds: Chemistry of Carbon Compounds, Vol. 1, Part C, p. 331 (1965)]. The compound having a halogenoacetyl amino group can be easily produced by applying the known amidation process [cf. R. B. Wagner, H. D. Zook: Synthetic Organic Chemistry, p. 566 (1955)] or any process analogous thereto, to such an amino-containing compound.

## Example 1

1.75 g of 7-amino-3-(N-chloroacetylcarbamoyloxymethyl)-3-cephem-4-carboxylic acid were suspended in 15 ml of water and the suspension was stirred. 0.42 g of sodium hydrogen carbonate were added to obtain a solution. After adding 1.1 g of sodium N-methyldithiocarbamate, the mixture was stirred at room temperature for 1.5 hours. The crystals were collected by filtration, rinsed with water and dried. Recrystallization from diethyl ether gave crystals of N-methylrhodanine, m.p. 71—72°C (literature 72°C, Anlreash: Monatshefte für Chemie *25*, 167). The first filtrate after filtration of N-methylrhodanine was adjusted to pH 3.5 with N-hydrochloric acid and allowed to stand under ice-cooling for one hour. The precipitate was collected by filtration, washed with water, methanol and

diethyl ether in the order mentioned and dried. The above procedure produced 1.06 g of 7-amino-3-carbamoyloxy-methyl-3-cephem-4-carboxylic acid as a white powder.

UV (pH 6.5, phosphate): $\lambda$ max 265nm ($\varepsilon$ 7885)

NMR ($d_6$-DMSO): 3.34, 3.57 ppm(2H, ABq, 2—$CH_2$), 4.58, 4.87 ppm (2H, ABq, 3—$CH_2$), 4.76ppm (1H, d, 6—H), 4.96 ppm (1H, d, 7—H), 6.50 ppm(2H, bs, —$OCONH_2$)

Elemental analysis, for $C_9H_{11}N_3O_5S.0.5H_2O$

Calcd. C, 38.29; H, 4.29; N, 14.89

Found C, 38.84; H, 4.25; N, 14.12

## Example 2

1.6 g of 7-[2-(2-chloroacetamidothiazol-4-yl)-2-(*syn*)-methoxyiminoacetamido]-3-(N-chloroacetylcarbamoyloxymethyl)-3-cephem-4-carboxylic acid were suspended in 10 ml of water, and with stirring, sodium hydrogen carbonate was added to adjust the pH to 7.0. Then, after addition of 0.80 g of sodium N-methyldithiocarbamate, the mixture was stirred at room temperature for one hour. The reaction mixture was washed with ethyl acetate and the water layer was taken and purified by column chromatography on Amberlite XAD-2 (Trade Mark, Rohm & Haas Co.). 650 mg of sodium 7-[2 - (2 - aminothiazol - 4 - yl) - 2 - (*syn*) - methoxyiminoacetamido] - 3 - carbamoyloxymethyl - 3-cephem - 4 - carboxylate were obtained as a white powder by the above procedure.

Elemental analysis, for $C_{15}H_{15}N_6O_7S_2Na.3H_2O$

Calcd. C, 33.84; H, 3.98; N, 15.78

Found C, 33.94; H, 3.82; N, 15.42

NMR(60MHz, $D_2O$): 3.47 ppm (2H, q, 2—$CH_2$), 3.92 ppm (3H, s, $OCH_3$), 4.68 ppm(2H, q, —$CH_2$—OCONH), 5.27 ppm(1H, d, 6—H), 5.72 ppm (1H, d, 7—H), 6.95 ppm(1H, s, thiazole 5—H)

## Example 3

164.5 g of 7-(5-carboxy-5-phthalimidovaleramido)-3-hydroxymethyl-3-cephem-4-carboxylic acid ditriethylamine salt were dissolved in 600 ml of dichloromethane. After the solution was cooled to an internal temperature of 0 to −5°C, a solution of 105 g of chloroacetyl isocyanate in 100 ml of dichloromethane was added dropwise. After the dropwise addition, the mixture was stirred for 30 minutes and 600 ml of water were added dropwise to the reaction mixture, followed by stirring for 20 minutes. The reaction mixture was separated into layers and the upper layer was taken. This procedure produced an aqueous solution of 7-(5-carboxy-5-phthalimidovaleramido)-3-(N-chloroacetyl-carbamoyloxymethyl)-3-cephem-4-carboxylic acid ditriethylamine salt.

64.1 g of sodium N-methyldithiocarbamate were added to this solution and the mixture was stirred at room temperature for one hour, after which it was washed with ethyl acetate and the water layer was taken. This aqueous solution was adjusted to pH 2.0 with phosphoric acid and extracted with a mixture of ethyl acetate and tetrahydrofuran. The extract was washed with saturated aqueous sodium chloride and dried over magnesium sulphate. The solvent was distilled off under reduced pressure and the viscous oily residue thus obtained was treated with diethyl ether. The powders thus obtained were dried under reduced pressure over phosphorus pentoxide. 126.5 g of 7-(5-carboxy-5-phthalimidovaleramido)-3-carbamoyloxymethyl-3-cephem-4-carboxylic acid were obtained by the above procedure.

NMR($d_6$—DMSO): 1.26—2.36 ppm(6H, m, —$(CH_2)_3$—), 3.45 ppm(2H, ABq,2—$CH_2$), 4.74 ppm(1H, t, —CH), 4.77 ppm (2H, ABq, 3—$CH_2$), 5.06 ppm (1H, d, 6—H), 5.62 ppm (1H, dd, 7—H), 6.56 (2H, s, $CONH_2$), 7.92 ppm (4H, s, aromatic ring-H)

## Example 4

254 g of 7-[5-carboxy-5-(*p-t*-butylbenzamido)valeramido]-3-hydroxymethyl-3-cephem-4-carboxylic acid monotriethylamine salt were suspended in 1 l of dichloromethane, and, with stirring, 20.1 g of triethylamine were added at room temperature to obtain a solution. The solution thus obtained was cooled to an internal temperature of 0 to −5°C and 96 g of chloroacetyl isocyanate dissolved in 100 ml of dichloromethane were added dropwise. After the dropwise addition had been completed, the mixture was stirred for 20 minutes, at the end of which 1 l of water and 20.1 g of triethylamine were added. The mixture was stirred for 10 minutes, after which it was separated into layers and the water layer was taken. The above procedure yielded an aqueous solution containing 7-[5-carboxy-5-(*p-t*-butylbenzamido)valeramido] - 3-(N-chloroacetylcarbamoyl-oxymethyl)-3-cephem-4-carboxylic acid di-triethylamine salt. 88 g of sodium N-methyldithiocarbamate were added to this solution and the mixture was stirred at room temperature for 1.5 hours. The reaction mixture was washed twice with 500 ml portions of ethyl acetate, followed by the addition of 500 ml of tetrahydrofuran and 500 ml of ethyl acetate. The mixture was adjusted to pH 2.0 with phosphoric acid and, after thorough shaking, the organic layer was taken, washed with saturated aqueous sodium chloride and dried over magnesium sulphate. The solvent was distilled off under reduced pressure, the viscous oily residue was treated with diethyl ether and the resulting powders were dried under reduced pressure over phosphorus pentoxide. The above procedure produced 169 g of 7-[5-carboxy-5-(*p-t*-butylbenzamido)valeramido]-3-carbamoyloxymethyl-3-cephem-4-carboxylic acid. A 5.7 g portion of the above product was taken, suspended in 15 ml of water and dissolved by the addition of 2.1 g of triethylamine. The solution was purified by column chromatography on Amberlite XAD-2 and Sephadex LH—20 (Trade Mark, Pharmacia Fine Chemicals Co.). The above procedure produced 2.6 g of 7-[5-carboxy-5-(*p*-*t*-butylbenzamido)valeramido]-3-carbamoyl-oxymethyl-3-cephem-4-carboxylic acid ditriethylamine salt.

Elemental analysis, for $C_{38}H_{62}N_6O_9S.H_2O$

Calcd. C, 57.26; H, 8.09; N, 10.54

Found C, 57.17; H, 8.41; N, 10.38

NMR($d_6$—DMSO): 1.13 ppm(18H, t, $N(CH_2CH_3)_3$), 1.31 ppm(9H, s, t—Bu), 1.4—2.0 ppm (4H, m, $(CH_2)_2$), 3.17, 3.48 ppm (2H, ABq, 2—$CH_2$),

4.24 (1H, m, —CH),

4.66, 4.87 ppm (2H, ABq, 3—$CH_2$), 4.94 ppm(1H, d, 6—H), 5.50 ppm(1H, dd, 7—H), 6.44 ppm(2H, bs, $CONH_2$), 7.46 ppm (2H, d, aromatic ring—H), 7.79 ppm (2H, d, aromatic ring—H)

## Example 5

9.73 g of 7-[2-(2-chloroacetamidothiazol-4-yl)-2-(*syn*)-methoxyiminoacetamido]-3-carbamoyloxymethyl-3-cephem-4-carboxylic acid were suspended in 50 ml of water, and dissolved by the addition of 1.55 g of sodium hydrogen carbonate with stirring. Then, after the addition of 2.6 g of sodium N-methyldithiocarbamate, the mixture was stirred at room temperature for one hour. The reaction mixture was washed twice with 50 ml portions of ethyl acetate and the water layer was degassed and purified by column chromatography on Amberlite XAD—2. The above procedure produced 5.1 g of sodium 7-[2-(2-aminothiazol-4-yl)-2-(*syn*)-methoxyiminoacetamido]-3-carbamoyloxymethyl-3-cephem-4-carboxylate as a white powder.

The product was identical with the compound obtained according to Example 2.

## Example 6

2.4 g of 7-[2-(2-chloroacetamidothiazol-4-yl)-2-(*syn*)-methoxyiminoacetamido]-cephalosporanic acid were suspended in 7 ml of water and, with stirring, 384 mg of sodium hydrogen carbonate were added.

650 mg of sodium N-methyldithiocarbamate were added to the solution thus obtained and the mixture was stirred at room temperature for one hour. The reaction mixture was washed with ethyl acetate and the water layer was purified by column chromatography on Amberlite XAD—2. 1.11 g of 7-[2-(2-aminothiazol-4-yl)-2-(*syn*)-methoxyiminoacetamido]cephalosporanic acid were obtained as a white powder by the above procedure.

Elemental analysis, for $C_{16}H_{16}M_5O_7S_2Na.2.5H_2O$

Calcd. C, 36.78; H, 4.05; N, 13.40

Found C, 36.93; H, 3.80; N, 12.68

NMR(60MHz, $D_2O$): 2.07 ppm(3H, s, $COCH_3$), 3.53 ppm(2H, q, 2—$CH_2$), 3.98 ppm(3H, s, $OCH_3$), 4.75 ppm(2H, q, 3—$CH_2$), 5.21 ppm(1H, d, 6—H), 5.81 ppm(1H, d, 7—H), 7.01 ppm(1H, s, thiazole 5—H)

## Example 7

1.6 g of 7-[2-(2-chloroacetamidothiazol-4-yl)-2-(*syn*)-methoxyiminoacetamido]-3-(1-methyl-1H-tetrazol-5-yl)thiomethyl-3-cephem-4-carboxylic acid were suspended in 6.5 ml of water, and dissolved by the addition of 250 mg of sodium hydrogen carbonate with stirring. Then, after the addition of 415 mg of sodium N-methyldithiocarbamate, the mixture was stirred at room temperature for one hour. The reaction mixture was washed with ethyl acetate and the water layer was purified by column chromatography on Amberlite XAD—2. The above procedure produced 714 mg of sodium 7-[2 - (2 - aminothiazol - 4 - yl) - 2 - (*syn*) - methoxyiminoacetamido] - 3 - (1 methyl - 1H - tetrazol - 5-yl)thiomethyl - 3 - cephem - 4 - carboxylate as a white powder.

Elemental analysis for $C_{16}H_{16}N_9O_5S_2Na.2H_2O$

Calcd. C, 33.74; H, 3.54; N, 22.13

Found C, 34.18; H, 3.57; N, 21.79

NMR(60 MHz, $D_2O$): 3.59 ppm(2H, q, 2—$CH_2$), 3.93 ppm(3H, s, $OCH_3$), 3.98 ppm(3H, s, N—$CH_3$), 4.18 ppm(2H, q, 3—$CH_2$), 5.12 ppm(1H, d, 6—H), 5.72 ppm(1H, d, 7—H), 6.93 ppm(1H, s, thiazole 5—H).

## Example 8

28.5 g of 7-[2-(2-chloroacetamidothiazol-4-yl)-2-(*syn*)-methoxyiminoacetamido]-desacetoxycephalosporanic acid were suspended in 250 ml of water, and then dissolved by the addition of 5.05 g of sodium hydrogen carbonate with stirring. Then, after the addition of 9.42 g of sodium N-ethyldithiocarbamate, the mixture was stirred at room temperature for 1.5 hours. The reaction mixture was washed with ethyl acetate and the water layer was purified by column chromatography on Amberlite

XAD—2. The above procedure produced 18.0 g of sodium 7-[2-(2-aminothiazol-4-yl)-2-(*syn*)-methoxyiminoacetamido]desacetoxycephalosporanate as a white powder.

Elemental analysis, for $C_{14}H_{14}N_5O_5S_2Na.1.5H_2O$

Calcd. C, 37.67; H, 3.84; N, 15.68

Found C, 37,37; H, 3.98; N, 15.38

NMR(60 MHz, $D_2O$): 1.94 ppm(3H, s, 3—$CH_3$), 3.46 ppm(2H, q, 2—$CH_2$), 4.00 ppm (3H, s, $OCH_3$), 5.17 ppm(1H, d, 6—H), 5.76 ppm(1H, d, 7—H), 6.99 ppm(1H, s, thiazole 5—H)

## Example 9

4.58 g of monosodium 7-(5-carboxy-5-aminovaleramido)-3-hydroxymethyl-3-cephem-4-carboxylate were dissolved in a mixture of 18 ml of water and 6 ml of tetrahydrofuran. While the solution was adjusted to pH 8.0-9.0 by the addition of 3N-aqueous sodium hydroxide, 6 ml of a tetrahydrofuran solution containing 2.12 g of *p*-*t*-butylbenzoyl chloride were added dropwise. After the dropwise addition had been completed, the mixture was further stirred for 30 minutes and, then, adjusted to pH 2.5 with phosphoric acid. The solution was extracted with a mixture of dichloromethane and tetrahydrofuran. The extract was washed with saturated aqueous sodium chloride and dried over magnesium sulphate. The solvent was distilled off and the residue was dissolved by the addition of 50 ml of dichloromethane and 2.8 ml of triethylamine. The solution was concentrated under reduced pressure, the residue was dissolved by the addition of 80 ml of dichloromethane and the solution was concentrated under reduced pressure. The above operation was repeated twice, whereupon 7 - [5 - carboxy - 5 - (*p* - *t* - butylbenzamido) - valeramido] - 3 - hydroxymethyl - 3 - cephem - 4-carboxylic acid ditriethylamine salt was obtained. This product was dissolved in 60 ml of dichloromethane containing 3.2 ml of triethylamine and the solution was cooled to an internal temperature of −5 to −10°C. With stirring, 10 ml of dichloromethane containing 3.0 g of chloroacetyl isocyanate were added dropwise. After the dropwise addition had been completed, the mixture was further stirred for 20 minutes to obtain a dichloromethane solution containing 7-[5-carboxy-5-(*p*-*t*-butylbenzamido)valeramido]-3-(N-chloroacetylcarbamoyloxymethyl)-3-cephem-4-carboxylic acid di-triethylamine salt. This reaction mixture was cooled to −20°C and 3.26 g of trimethylchlorosilane were added in a single dose. The mixture was stirred at −10°C for one hour. It was cooled to −30°C and 3 ml of N,N-dimethylaniline and 4.2 g of phosphorus pentachloride were added. The mixture was maintained at an internal temperature of −20 to −30°C. After stirring the mixture at that temperature for 2 hours, 37 ml of methanol were added dropwise at an internal temperature of no more than −30°C. After the dropwise addition had been completed, the mixture was stirred at −5°C for 25 minutes, at the end of which time 22 ml of water were added. The pH was adjusted to 3.5 with aqueous ammonia, whereupon 7-amino-3-(N-chloroacetylcarbamoyloxymethyl)-3-cephem-4-carboxylic acid separated. Without isolating the precipitate, the reaction mixture was adjusted to pH 6.5 by the addition of 3N-aqueous sodium hydroxide. Then, after the addition of 2 g of sodium N-methyldithiocarbamate, the mixture was stirred at room temperature for one hour. It was then adjusted to pH 3.5 with hydrochloric acid and allowed to stand under ice-cooling. The resulting precipitate was collected by filtration, washed with water and methanol, and dried. The above procedure produced 1.13 g of 7-amino-3-carbamoyloxymethyl-3-cephem-4-carboxylic acid as a white powder. This product was identical with the product obtained according to Example 1.

## Example 10

204 mg of *p*-nitro-N-chloro-acetylaniline were dissolved in a mixture of 7 ml of methanol and 10 ml of tetrahydrofuran.

180 mg of sodium N-methyl-dithiocarbamate were added to the solution and the mixture was stirred at room temperature for 2 hours. The reaction mixture was concentrated under reduced pressure and the residue was dissolved in ethyl acetate and extracted with 5% HCl. The extract was neutralized with sodium hydrogen carbonate and extracted with ethyl acetate. The organic layer was washed with water and dried over magnesium sulphate. The solvent was distilled off and the residue was recrystallized from diethyl ether. The above procedure produced 105 mg of *p*-nitroaniline; m.p. 145—146°C.

## Example 11

1 g of 2-(2-chloroacetamidothiazol-4-yl)-2-(*syn*)-methoxyiminoacetic acid was dissolved in 16 ml of tetrahydrofuran, followed by the addition of 0.93 g of sodium N-methyl-dithiocarbamate. The mixture was stirred at room temperature for 2 hours. The precipitate was collected by filtration and washed with a small amount of tetrahydrofuran, followed by the addition of 15 ml of water. The precipitate dissolved once and crystals were then formed. These crystals were collected by filtration (0.7 g) and recrystallized from aqueous ethanol. The above procedure produced 2-(2-amino-thiazol-4-yl)-2-(*syn*)-methoxyiminoacetic acid as colourless needles, m.p. 164.6°C.

Elemental analysis, for $C_6H_7N_3O_3S.1.5H_2O$

Calcd. C, 31.57; H, 4.41; N, 18.41

Found C, 31.44; H, 4.32; N, 18.35

NMR spectrum (90 MHz, $D_2O$ containing $NaHCO_3$): 3.96 ppm(s, 3H, $OCH_3$), 6.91 ppm(s, 1H, thiazole 5—H)

Example 12

2-(2-chloroacetamidothiazol-4-yl)-2-(*anti*)-methoxy-iminoacetic acid was reacted in the same manner as in Example 11 to obtain 2-(2-aminothiazol-4-yl)-2-(*anti*)-methoxyiminoacetic acid as pale yellow crystals, m.p. 168.0°C.

Elemental analysis, for $C_6H_7N_3O_3S$

Calcd. C, 35.81; H, 3.50; N, 20.88

Found C, 35.71; H, 3.40; N, 20.83

NMR spectrum (90 MHz, $D_2O$ containing $NaHCO_3$): 4.03 ppm(s, 3H, $OCH_3$), 7.46(s, 1H, thiazole 5—H)

Example 13

640 mg of benzhydryl 7-(2-thienylacetamido)-3-(N-chloroacetylcarbamoyloxymethyl)-3-cephem-4-carboxylate were dissolved in 5 ml of tetrahydrofuran, followed by the addition of 260 mg of sodium N-methyldithiocarbamate dissolved in 2 ml of water. The mixture was stirred at room temperature for 4 hours. A major portion of the tetrahydrofuran was distilled off and the residue was extracted with chloroform. The chloroform layer was washed with water, dried and concentrated. The residue was chromatographed on silica gel, elution being carried out with chloroform-ethyl acetate (2:1). The above purification procedure produced benzhydryl 7-(2-thienyl-acetamido)-3-carbamoyloxy-methyl-3-cephem-4-carboxylate as a white powder.

Elemental analysis, for $C_{28}H_{25}N_3O_6S_2$

Calcd. C, 59.67; H, 4.47; N, 7.45

Found C, 59.10; H, 4.34; N, 7.32

NMR spectrum (60 MHz, $d_6$-DMSO-deuteriochloroform); 3.50 ppm(bs. 2H, 2—$CH_2$), 3.86 ppm(s, 2H, 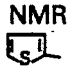 $CH_2$), 4.95 ppm(bs, 2H,

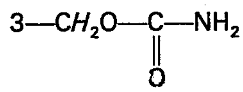

**Claims**

1. A method for removing a halogenoacetyl group from a halogenoacetyl amino compound, characterised in that a halogenoacetyl amino compound is reacted with an N-substituted dithiocarbamic acid having the formula

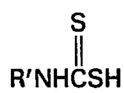

(wherein R′ is an organic group) or a salt thereof.

2. A method as claimed in Claim 1, wherein the organic group represented by R′ is an alkyl group having 1 to 4 carbon atoms or a cycloalkyl group having 5 to 7 carbon atoms.

3. A method as claimed in Claim 1 or 2, wherein the halogenoacetyl amino compound is a cephem compound.

4. A method as claimed in any one of Claims 1 to 3, wherein the halogenoacetyl amino compound is a compound of the formula:

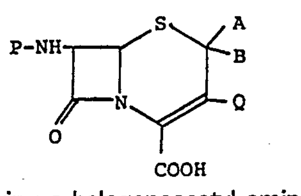

wherein P is an organic acyl group having a halogenoacetyl amino group, each of A and B is hydrogen or methyl, and Q is hydrogen, methyl, acetoxymethyl, carbamoyloxymethyl, (1-methyl-1H-tetrazol-5-yl)thiomethyl, 5-acetylamino-1,3,4-thiadiazol-2-yl,
or a pharmaceutically acceptable salt thereof.

5. A method as claimed in Claim 3, wherein 7-[2-(2-chloroacetamidothiazol-4-yl)-2-(*syn*)-methoxyiminoacetamido]-3-(1-methyl-1H-tetrazol-5-yl)thiomethyl-3-cephem-4-carboxylic acid or an alkali metal or amine salt thereof is reacted with an N-methyl (or ethyl)-dithiocarbamic acid alkali metal salt.

6. A method as claimed in Claim 3, wherein 7-[2-(2-chloroacetamidothiazol-4-yl)-2-(*syn*)-methoxyiminoacetamido]-3-(1-methyl-1H-tetrazol-5-yl)thiomethyl-3-cephem-4-carboxylic acid sodium salt is reacted with sodium N-methyldithiocarbamate.

7. A method as claimed in Claim 1, wherein 2-(2-chloroacetamidothiazol-4-yl)-2-(*syn*)-methoxy-

iminoacetic acid or an alkali metal or amine salt thereof is reacted with an N-methyl (or ethyl)dithiocarbamic acid alkali metal salt.

8. A method as claimed in Claim 1, wherein 2-(2-chloroacetamidothiazol-4-yl)-2-(*syn*)-methoxyiminoacetic acid is reacted with sodium N-methyldithiocarbamate.

9. A method as claimed in any one of Claims 1 to 8, wherein the reaction is carried out in an inert solvent at a temperature between —10°C and 50°C.

**Revendications**

1. Procédé pour éliminer par scission un groupe halogénoacétyle d'un composé halogénoacétyl-aminé, caractérisé en ce qu'on fait réagir un composé halogénoacétyl-aminé avec un acide dithiocarbamique N-substitué ayant la formule

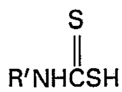

(dans laquelle R' est un groupe organique) ou un sel d'un tel acide.

2. Procédé selon la revendication 1, caractérisé en ce que le groupe organique représenté par R' est un groupe alcoyle ayant de 1 à 4 atomes de carbone ou un groupe cycloalcoyle ayant de 5 à 7 atomes de carbone.

3. Procédé selon l'une des revendications 1 et 2, caractérisé en ce que le composé halogénoacétyl-aminé est un composé "cephem".

4. Procédé selon l'une des revendications 1 à 3, caractérisé en ce que le composé halogénoacétyl-aminé est un composé de la formule:

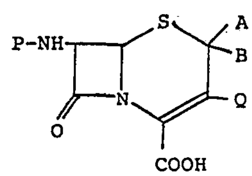

dans laquelle P est un groupe organique acyle ayant un groupe halogénoacétyl-amino, A et B sont chacun de l'hydrogène ou un groupe méthyle et Q est de l'hydrogène, un groupe méthyle, acétoxyméthyle, carbamoyloxyméthyle, (1-méthyl-1H-tétrazol-5-yl)thiométhyle, 5-acétylamino-1,3,4-thiadiazol-2-yle ou un sel pharmaceutiquement acceptable d'un tel composé.

5. Procédé selon la revendication 3, caractérisé en ce qu'on fait réagir l'acide 7-[2-(2-chloroacétamidothiazol-4-yl)-2-(*syn*)-méthoxyiminoacétamido]- 3-(1-méthyl-1H-tétrazol-5-yl)thiométhyl-3-céphem-4-carboxylique ou un sel de métal alcalin ou d'amine de cet acide avec un sel de métal alcalin d'acide N-méthyl (ou éthyl) dithiocarbamique.

6. Procédé selon la revendication 3, caractérisé en ce qu'on fait réagir le sel de sodium de l'acide 7-[2-(2-chloroacétamidothiazo-4-yl)-2-(*syn*)-méthoxyiminoacétamido]- 3-(1-méthyl-1H-tétrazol-5-yl)-thiométhyl-3-céphem-4-carboxylique avec le N-méthyldithiocarbamate de sodium.

7. Procédé selon la revendication 1, caractérisé en ce qu'on fait réagir l'acide 2-(2-chloroacétamidothiazol-4-yl)-2-(*syn*)-méthoxyiminoacétique ou un sel de métal alcalin ou d'amine de cet acide avec un sel de métal alcalin d'acide N-méthyl (ou éthyl) dithiocarbamique.

8. Procédé selon la revendication 1, caractérisé en ce qu'on fait réagir l'acide 2-(2-chloroacétamidothiazol-4-yl)-2-(*syn*)-méthoxyiminoacétique avec le N-méthyldithiocarbamate de sodium.

9. Procédé selon l'une quelconque des revendications 1 à 8, caractérisé en ce que la réaction est conduite dans un solvant inerte à une température comprise entre —10°C et 50°C.

**Patentansprüche**

1. Verfahren zur Entfernung einer Halogenacetyl-Gruppe aus einer Halogenacetyl-amino-Verbindung, dadurch gekennzeichnet, daß die Halogenacetyl-amino-Verbindung mit einer N-substituierten Dithiocarbaminsäure der Formel

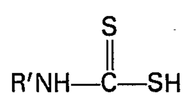

worin R' eine organische Gruppe bezeichnet, oder einem ihrer Salze zur Reaktion gebracht wird.

2. Verfahren nach Anspruch 1, dadurch gekennzeichnet, daß die durch R' bezeichnete organische Gruppe eine Alkyl-Gruppe mit 1 bis 4 Kohlenstoff-Atomen oder eine Cycloalkyl-Gruppe mit 5 bis 7 Kohlenstoff-Atomen ist.

3. Verfahren nach Anspruch 1 oder 2, dadurch gekennzeichnet, daß die Halogenacetyl-amino-Verbindung eine Cephem-Verbindung ist.

4. Verfahren nach Anspruch 1 bis 3, dadurch gekennzeichnet, daß die Halogenacetyl-amino-Verbindung eine Verbindung der Formel

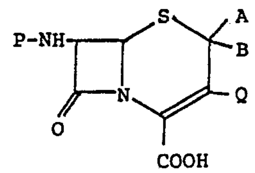

worin P für eine organische Acyl-Gruppe mit einer Halogenacetyl-amino-Gruppe, sowohl A als auch B für ein Wasserstoff-Atom oder eine Methyl-Gruppe und Q für ein Wasserstoff-Atom oder die Gruppe Methyl, Acetoxymethyl, Carbamoyloxymethyl, (1-Methyl-1H-tetrazol-5-yl)-thiomethyl bzw. 5-Acetyl-amino-1,3,4-thiadiazol-2-yl steht, oder eines ihrer pharmazeutisch unbedenklichen Salze ist.

5. Verfahren nach Anspruch 3, dadurch gekennzeichnet daß 7[2-(2-Chloracetamidothiazol-4-yl)-2-(syn)-methoxyiminoacetamido]-3-(1-methyl-1H-tetrazol-5-yl)-thiomethyl-3-cephem-4-carbonsäure oder eines ihrer Alkalimetall- oder Amin-Salze mit N-Methyl (oder Ethyl)-dithiocarbaminsäure-alkalimetallsalz zur Reaktion gebracht wird.

6. Verfahren nach Anspruch 3, dadurch gekennzeichnet, daß 7-[2-(2-Chloracetamidothiazol-4-yl)-2 - (syn) - methoxyiminoacetamido] - 3 - (1 - methyl - 1H - tetrazol - 5 - yl) - thiomethyl - 3 - cephem-4 - carbonsäure - natriumsalz mit Natrium - N - methyl - dithiocarbamat zur Reaktion gebracht wird.

7. Verfahren nach Anspruch 1, dadurch gekennzeichnet, daß 2-(2-Chloracetamidothiazol-4-yl)-2-(syn)-methoxyiminoessigsäure oder eines ihrer Alkalimetall- oder Amin-Salze mit N-Methyl (oder Ethyl)-dithiocarbaminsäure-alkalimetallsalz zur Reaktion gebracht wird.

8. Verfahren nach Anspruch 1, dadurch gekennzeichnet, daß 2-(2-Chloracetamidothiazol-4-yl)-2-(syn)-methoxyiminoessigsäure mit Natrium-N-methyl-dithiocarbamat zur Reaktion gebracht wird.

9. Verfahren nach Anspruch 1 bis 8, dadurch gekennzeichnet, daß die Reaktion in einem inerten Lösungsmittel bei einer Temperatur zwischen —10°C und 50°C durchgeführt wird.